# FASCICULE DE BREVET EUROPEEN

(11) **EP 3 518 940 B1**
(45) Date de publication et mention de la délivrance du brevet: **22.11.2023**
(21) Numéro de dépôt: 17784369.5
(22) Date de dépôt: 26.09.2017
(51) Int. Cl.: A61K 31/737, A61K 35/08, A61K 36/03, A61K 9/08, A61P 29/00, A61P 37/04

(54) **COMPOSITION AQUEUSE DÉRIVÉE D'EAU DE MER ET D'ALGUES MARINES**
AUS MEERWASSER UND ALGEN GEWONNENE, WÄSSRIGE ZUSAMMENSETZUNG
AQUEOUS COMPOSITION DERIVED FROM SEAWATER AND SEAWEED

(30) Priorité: 03.10.2016 FR 1659484
(43) Date de publication de la demande: 07.08.2019
(73) Titulaire: YS Lab, 29000 Quimper (FR)
(72) Inventeur: FAGON, Roxane, 29140 Saint Yvi (FR); DUTOT-WOLF, Melody, 94210 La Varenne Saint Hilaire (FR); TANTER, Caroline, 29000 Quimper (FR); HEMON, Marc, 22490 Plouer sur Rance (FR)
(74) Mandataire: Ipside
(86) Numéro de dépôt international: PCT/FR2017/052590
(87) Numéro de publication internationale: WO 2018/065697

(56) Documents cités:
- WO-A1-2015/071497
- WO-A1-2015/082356
- US-B1- 6 921 540
- ZEDONG JIANG ET AL: "The potent activity of sulfated polysaccharide, ascophyllan, isolated fromto induce nitric oxide and cytokine production from mouse macrophage RAW264.7 cells: Comparison between ascophyllan and fucoidan", NITRIC OXIDE: BIOLOGY AND CHEMISTRY, vol. 25, no. 4, 17 octobre 2011 (2011-10-17), pages 407-415, XP028120417, ISSN: 1089-8603, DOI: 10.1016/J.NIOX.2011.10.001 [extrait le 2011-10-17]
- R Sivangala ET AL: "Cytokines that Mediate and Regulate Immune Responses", Innovative Immunology, 15 avril 2015 (2015-04-15), pages 1-26, XP055440385, Extrait de l'Internet: URL:http://austinpublishinggroup.com/ebook s/innovative-immunology/chapters/II-14-05. pdf [extrait le 2018-01-12]

## Description

La présente invention concerne une composition à base d'eau de mer, destinée en particulier à la stimulation du système immunitaire en vue de prévenir des agressions extérieures.

Les défenses physiques de l'organisme sont la peau, les muqueuses, les cils et villosités des cellules ciliées ainsi que les mucus et les fluides qui tapissent la plupart des muqueuses. Ces défenses permettent de bloquer mécaniquement la pénétration d'un agent exogène dans l'organisme et/ou de favoriser son évacuation, notamment par le biais du battement ciliaire.

Les défenses chimiques et cellulaires de l'organisme font intervenir plusieurs acteurs en plusieurs étapes. Dans un premier temps au début de l'infection, la réponse inflammatoire se déclenche de façon non spécifique pour activer les phagocytes et les macrophages qui vont digérer l'agent pathogène après sécrétion de cytokines et autres médiateurs de l'inflammation. Dans un second temps, si la première ligne de défense n'a pas suffi à éliminer l'agent pathogène, les leucocytes aidés des macrophages initient la réponse immunitaire pour reconnaître spécifiquement l'agent pathogène et le digérer. Dans certains cas, l'organisme n'arrive pas à lutter contre l'invasion et une pathologie se développe. Au niveau de la sphère oto-rhino-laryngologique, en cas de défaillance du système immunitaire, des rhinites, sinusites et laryngites peuvent se développer.

Les premières recommandations pour maintenir le système immunitaire réactif en cas d'agression sont de maintenir une hygiène de vie saine : ne pas fumer, faire de l'exercice régulièrement, limiter sa consommation d'alcool, dormir suffisamment, se laver les mains fréquemment... L'alimentation joue également un rôle dans le maintien du système immunitaire ; pour cette raison, il existe sur le marché des compléments alimentaires destinés à « booster le système immunitaire ». Il s'agit principalement de compléments alimentaires à base de vitamines (A, B, C, D et E) et/ou d'oligo-éléments (sélénium, zinc). Néanmoins, aucune étude suffisamment exhaustive ne démontre l'efficacité de tels compléments alimentaires. D'autres compléments alimentaires à base de probiotiques permettent d'améliorer le fonctionnement du système digestif et
ainsi l'assimilation des nutriments essentiels à la synthèse des vitamines (B et K surtout), mais aussi de restaurer la flore commensale.

En termes de prévention, pour bloquer le développement de certaines infections graves face auxquelles l'organisme ne peut pas lutter, la médecine propose la vaccination. La vaccination anti-infectieuse consiste à introduire un antigène dérivé ou similaire à l'agent infectieux, pour inciter l'organisme à déclencher une réponse immunitaire. Les vaccins peuvent être des virus atténués ou inactivés (pour éviter la pathologie virale), des bactéries ou encore des sous-unités antigéniques. La réaction immunitaire ainsi déclenchée permet de mémoriser l'antigène pour permettre à l'immunité acquise, lors d'une contamination vraie, de s'activer de façon plus rapide.

Par ailleurs, des solutions sont connues mettant en oeuvre des polysaccharides sulfatés et plus particulièrement des fucanes pour le traitement des pathologies faisant intervenir les défenses du système immunitaire. De tels fucanes sont décrits dans la demande WO2010/133359. La demande WO2010/133359 décrit l'utilisation de fucanes impliqués dans le bon fonctionnement du système immunitaire avec un impact positif sur la santé d'une flore intestinale de foetus pendant la gestation.

La demande WO 2015/082356 divulgue des compositions comprenant de l'eau de mer filtrée et un polysaccharide sulfaté contenu dans une algue. Ces compositions sont utiles pour le traitement des affections liées aux voies respiratoires et/ou aux muqueuses respiratoires.

Il existe un réel besoin en l'état d'améliorer les résultats obtenus grâce aux fucanes par exemple en activant leur effet grâce à une synergie d'action avec d'autres éléments. Pour combler les manquements de l'art antérieur, le but de la présente invention est de proposer une composition qui permette d'intervenir en amont avant l'entrée et/ou la prolifération des agents pathogènes en stimulant l'immunité innée.

L'invention est telle que définie dans les revendications 1-9.

L' objet de la présente invention concerne une composition aqueuse pour son utilisation dans la prévention d' moins une des maladies provoquées par l'invasion d'un ou plusieurs agents pathogènes par la peau, l'œil ou une muqueuse comprenant de 20 à 60% d'eau de mer filtrée, donné en pourcentage pondéral par rapport à la masse totale de la composition et comprenant un polysaccharide sulfaté contenu dans une algue marine, caractérisée en ce que la composition stimule le système immunitaire en stimulant l'immunité innée.

Selon l'invention, le polysaccharide sulfaté est un fucane ayant une masse de 2 à 21 KDa et provient de l'algue brune *Ascophyllum nodosum.*

Dans le cadre de l'invention l'expression « contenu dans une algue marine » signifie que la composition comporte soit tout ou partie de l'algue marine, soit que la composition comporte un extrait d'algue comprenant ledit polysaccharide sulfaté, soit encore que le polysaccharide sulfaté utilisé est une molécule de synthèse qui est identique à la molécule naturelle contenue dans ladite algue marine.

Dans le cadre de l'invention l'expression « obtenue à partir d'eau de mer » signifie que la composition comporte de l'eau de mer, ou bien qu'elle contient de l'eau de mer transformée.

L'immunostimulation a pour but de renforcer l'immunisation naturelle. Les macrophages, première ligne de défense dans la réponse immunitaire, ont un rôle dichotomique : ils déclenchent un processus inflammatoire qui doit être ensuite inhibé pour permettre le retour à une situation physiologique. Ces effets en apparence contradictoires reflètent la complexité de la réaction immunitaire. Il est donc tout à fait possible qu'un actif soit immunostimulant et anti-inflammatoire, tout dépend des mécanismes mis en jeu. De tels effets ont d'ailleurs été mis en évidence dans le cadre de l'étude de *Curcuma longa :* Chandrasekaran et al. Pharmacognosy Res.. 2013 April ; 5(2) : 71-79. Cela a pu être observé également dans le cadre d'extrait d'ail : lshiwada et al. J. Nutr. 2006;136 (3 Suppl ): 816S-820S ; Keiss et al., J. Nutr. 2003;133 (7): 2171-2175. De même, dans le cadre de l'étude de la *quercetine* : Liao et al. J. Agric. Food. Chem. 2014 Apr 2; 62(13):2872-80.

Les inventeurs ont montré de manière inattendue que la composition selon l'invention a des effets immunostimulants en augmentant la sécrétion de peptide antimicrobien qualifié de β-défensine-2, en augmentant la capacité d'autophagie des cellules, et en augmentant la sécrétion d'IL-10. La composition a également des propriétés anti-inflammatoires en inhibant la sécrétion de TNFα et d'IL-1β stimulée par le LPS d'E. Coli. En outre, il est intéressant de noter qu'en l'absence de stress inflammatoire bactérien (induit par le LPS), la composition selon l'invention stimule la réponse immunitaire en augmentant légèrement la sécrétion de TNFα et d'IL-1β (voir les bâtons noirs des diagrammes 4 et 5).

Le tableau 1 montre les éléments constitutifs de l'eau de mer ainsi que leur proportion théorique, exprimés en parts par million :

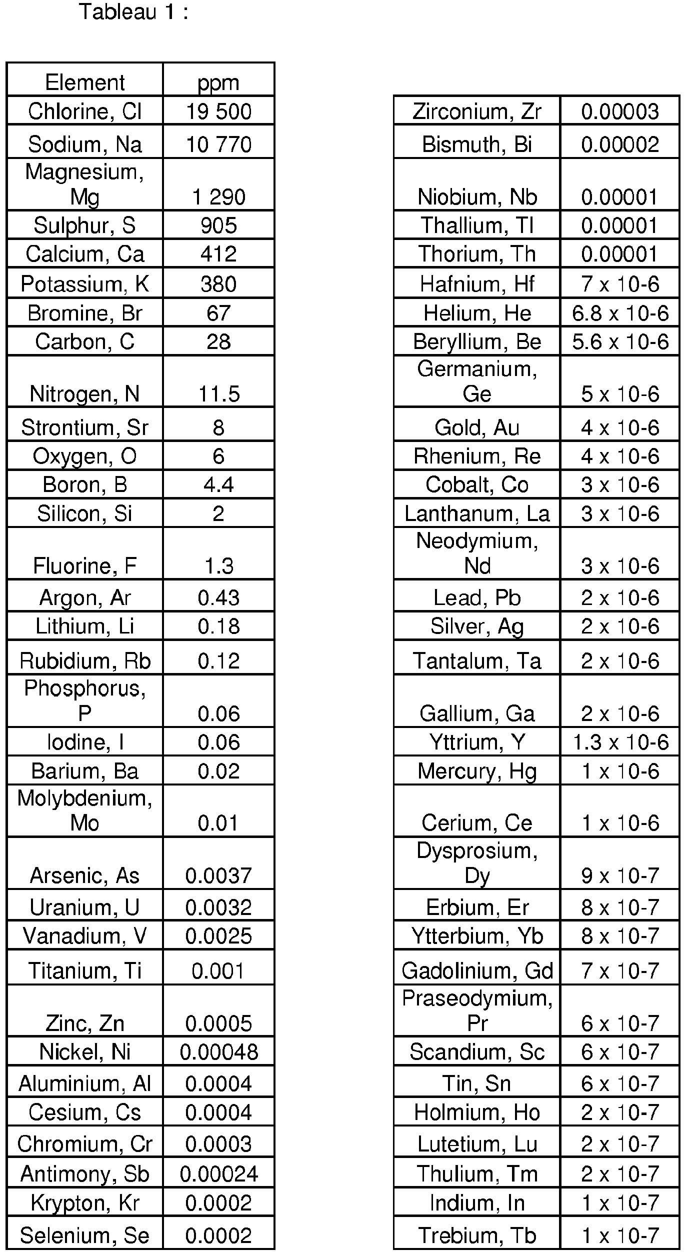

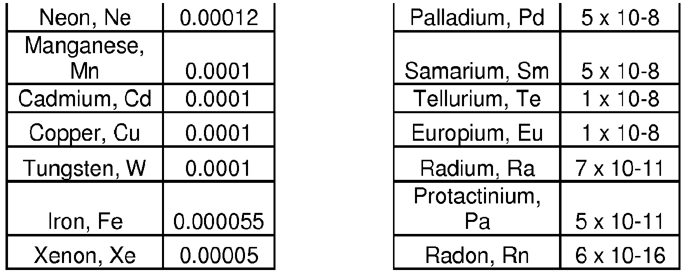

Les concentrations exprimées dans le tableau 1, le sont en valeurs moyennes, qui peuvent varier selon la localisation géographique de la mer, et même du lieu de pompage de l'eau de mer.

L'eau de mer à la base de la composition selon l'invention, constitue un réservoir de différents éléments chimiques, présents en concentration plus ou moins importante. Les propriétés physico-chimiques de l'eau de mer sont dues à un équilibre entre anions et cations. Ces éléments se comportent comme un ensemble de biomolécules interagissant les unes avec les autres. Dans le cadre de l'invention, la synergie d'action se fait non seulement entre ces différents ions, mais également avec les polysaccharides sulfatés présents dans la composition.

Les inventeurs ont découvert de manière tout à fait inattendue qu'une telle composition intégrant une combinaison d'ingrédients issus de produits de la mer, lesquels ingrédients proviennent à la fois des substances dissoutes dans une eau de mer et d'algues qui voient leur croissance s'effectuer dans une eau de mer, permettait de prévenir le développement des maladies liées à l'incorporation et à la prolifération d'agents pathogènes dans l'organisme. Les pathogènes visés sont principalement des virus ou des bactéries ou des agents allergènes. La composition selon l'invention est destinée à un traitement à visée prophylactique pour agir en amont en aidant l'organisme à se prémunir des effets néfastes de tels agents pathogènes en stimulant l'immunité innée.

La présente composition selon l'invention stimule l'immunité innée, c'est-à-dire qu'elle permet une activation des défenses contre les agents pathogènes tels que ceux visés précédemment, sans que le corps du sujet ait été nécessairement mis en contact avec lesdits agents au préalable.

Il est démontré que la composition selon l'invention est particulièrement avantageuse de par le fait qu'une réponse biologique est induite dès la première application. Un effet in vitro est observé sur des cellules vivantes seulement après une heure de mise en contact.

De plus, l'effet mesuré est mémorisé par les cellules et reste rémanent même après élimination de la solution. Par conséquent, l'action biologique est donc préventive.

En outre, l'action biologique est observée pour différents types cellulaires, ce qui se traduit par une action possible à différents endroits de l'organisme par une application locale directement au niveau des cellules. L'action de préparation de l'organisme à se défendre se fait par différents mécanismes complémentaires permettant une protection vis-à-vis de différents types d'agents pathogènes (bactéries, virus, allergènes, micro-organismes fongiques).

La composition selon l'invention comprend 20 à 60%, et de préférence 35 à 55%, voire 40 à 50% d'eau de mer filtrée donné en pourcentage pondérale par rapport à la masse totale de la composition finale obtenue. L'eau de mer filtrée est une eau de mer traitée en effectuant un passage au travers d'au moins un filtre décontaminant ; le diamètre des pores dudit filtre est avantageusement inférieur à 1µm.

L'eau de mer est avantageusement une eau de mer bretonne sélectionnée de préférence dans la commune de Penmarch dans le département du Finistère, dans la baie de Saint-Brieuc dans le département des Côtes-D'armor, ou encore dans la baie de Saint-Malo dans le département d'Ille et vilaine.

L'eau de mer utilisée est sélectionnée pour son emplacement privilégié, qui conditionne et/ou impose :
- une absence d'industries à proximité avec un environnement sain ;
- un site de prélèvement non enclavé : renouvellement constant d'eau ;
- des courants sur la zone favorisant le renouvellement de l'eau ; et
- une eau de qualité reconnue satisfaisante par des organismes spécialisés, tels que l'IFREMER et la DDASS (direction départementale des affaires sanitaires et sociales).

La composition selon l'invention comprend avantageusement au moins une eau sélectionnée parmi de l'eau purifiée, de l'eau distillée et de l'eau déminéralisée dans une teneur de 40 à 80%, de préférence 45 à 65, voire de 50 à 60%. La pureté de l'eau de dilution utilisée pour ajuster les concentrations est étroitement contrôlée pour éviter l'adjonction d'éléments indésirables qui peuvent polluer ou modifier les propriétés physico-chimiques du milieu.

Selon l'invention, le polysaccharide sulfaté est un fucane. Une définition des fucanes est donnée dans la demande WO1999032099.

Selon l'invention, le polysaccharide sulfaté est extrait d'une algue brune.

Comme algue brune sont connues les espèces : *Ascophyllum nodosum, Fucus vesiculosus, et Undaria pinnatifida.*

Selon l'invention, l'algue brune est *Ascophyllum nodosum.* Les polysaccharides sulfatés extraits d'*Ascophyllum nodosum* ont démontré une forte synergie d'action en combinaison avec les éléments contenus dans l'eau de mer pour la stimulation du peptide antimicrobien β-défensines-2, impliqué dans l'immunité innée qui peut être considérée comme une première ligne de défense de l'organisme contre les attaques microbiennes.

Le polysaccharide sulfaté est avantageusement pris dans une teneur de 0.01 à 0.5%, de préférence 0.05 à 0.4%, voire de 0.09 à 0.25%.

Le polysaccharide sulfaté a avantageusement une masse de 2 à 21 kDa (kilodalton), de préférence 4 à 17.5 kDa, voire 5 à 15 kDa. Les tests ont démontré que parmi les polysaccharides et en particulier les fucanes, ceux de 4, 8 et 20 kDa permettaient d'obtenir une très bonne activité antimicrobienne. Les meilleurs résultats ont été obtenus avec des fucanes de 8 kDa.

Avantageusement, la concentration des éléments dans la composition finale, présents sous forme ionique, est telle que :

| | |
|---|---|
| Cl (g/L) | 5 - 15 |
| Na (g/L) | 2.5 - 7 |
| Mg (g/L) | 0.45 - 0.70 |
| Ca (g/L) | 0.10 - 0.30 |
| K (g/L) | 0.10 - 0.30 |

Avantageusement, la concentration des éléments et des substances dans la composition finale selon l'invention est telle que :

| | |
|---|---|
| CI (g/L) | 7.9 - 10.1 |
| Na (g/L) | 4.3 - 5.3 |
| Mg (g/L) | 0.51 - 0.63 |
| Ca (g/L) | 0.16 - 0.22 |
| K (g/L) | 0.15 - 0.23 |
| Sucres totaux (g/L) | 0.25 - 0.55 |

Avantageusement la teneur des substances suivantes dans la composition selon l'invention est telle que :
Sulfates totaux (g/Kg) 1.0 - 1.8

Les concentrations dans la composition utilisée pour une action biologique sont faibles pour une forte réponse cellulaire, ce qui traduit un coefficient d'efficacité élevé de ce complexe naturel marin.

La composition montre une totale innocuité envers les patients aux concentrations décrites, la rendant utilisable dans la population générale mais aussi pédiatrique.

La composition a également montré une parfaite tolérance pour les muqueuses, mêmes les plus délicates comme l'épithélium cilié de la muqueuse nasale. En effet, l'étude de la cytotoxicité de la composition selon l'invention a permis d'évaluer sa haute tolérance pour les utilisations visées. Différents tests d'évaluation de la cytotoxicité sur plusieurs types de cellules ainsi qu'une étude de tolérance chez l'homme ont été réalisés pour cerner au mieux la tolérance de la composition selon l'invention par l'organisme. Une étude d'observation chez des sujets sains illustre cette tolérance en respectant le fonctionnement normal des cellules ciliées de la muqueuse nasale.

Avantageusement, le pH de la composition est de 6.5 à 8.5. De préférence, le pH est de 6.9 à 8.1.

Avantageusement, l'osmolalité mesurée à 25° C est de 400 à 550 mOsmol/Kg. De préférence, l'osmolalité est de 450 à 500 mOsmol/Kg.

L'osmolalité est mesurée selon la méthode décrite dans la Pharmacopée Européenne 9.0 (neuvième édition) - point 2.2.35. La conductivité est mesurée selon la méthode décrite dans la Pharmacopée Européenne 9.0 - point 2.2.38.

La stimulation de l'immunité de la composition selon l'invention consiste avantageusement en une stimulation de l'immunité innée.

La, ou les, muqueuse(s) ciblées par le traitement préventif selon l'invention est, ou sont, sélectionnée(s) parmi la muqueuse nasale, la muqueuse buccale et la muqueuse vaginale. Avantageusement, la composition selon l'invention vise la prévention des états inflammatoires des muqueuses.

La composition selon l'invention peut s'appliquer à tout domaine faisant intervenir la physiologie cellulaire, dans les organes, tel que l'œil ou tels que les organes de la sphère ORL - notamment les muqueuses nasales - en vue de prévenir les affections telles que les rhinites, les congestions nasales, les sécheresses nasales, les sinusites ou les bronchites. La composition selon l'invention peut également s'appliquer à la peau et aux muqueuses vaginales, notamment dans des préparations pour l'hygiène intime.

La présente demande concerne aussi un procédé de préparation d'une composition telle que décrite précédemment dans le cadre de l'invention, comprenant les étapes :
a) pomper l'eau de mer ;
b) prétraiter éventuellement l'eau de mer à l'aide d'un premier filtre naturel ou artificiel ;
c) filtrer l'eau de mer éventuellement prétraitée au travers d'un deuxième filtre dont le diamètre moyen des pores est inférieur ou égal 5 µm ;
d) effectuer une autre filtration au travers d'un troisième filtre dont le diamètre moyen des pores est inférieur à ceux du deuxième filtre et est inférieur ou égal 0.45 µm ;
e) diluer la solution filtrée avec de l'eau purifiée.

A l'étape a), l'eau de mer prélevée par pompage est filtrée éventuellement (étape b)) à l'aide, soit d'un filtre naturel comme par exemple du sable, soit d'un filtre artificiel comme par exemple une grille adaptée pour retirer les particules de grosse taille. L'eau est ensuite avantageusement entreposée dans un bassin ou une cuve de prétraitement. Cette eau de mer est ensuite redirigée vers des canalisations à l'aide d'une pompe en vue de sa filtration.

L'eau de mer prétraitée est filtrée aux étapes c) et d) au travers de deux filtres dont les diamètres moyens sont respectivement de 5 µm à 1 µm (pour c)), et inférieur à 0,45 µM (pour d)). Pour cette étape de filtration, l'eau est de préférence introduite dans un ensemble de filtration intégrant des canalisations qui comportent avantageusement des filtres sous forme de cartouches cylindriques. Un exemple d'ensemble de filtration est décrit à la suite dans la partie expérimentale.

La présente invention sera mieux comprise à la lecture de la partie expérimentale qui va suivre, dont les exemples et les figures sont uniquement donnés à titre illustratif et ne peuvent nullement être considérés comme limitatifs. Pour illustrer les résultats de la partie expérimentale, le schéma 1 représente une vue schématique en coupe transversale d'un ensemble de filtration qui permet de filtrer l'eau de mer avant sa dilution pour obtenir la composition selon l'invention.

### PARTIE EXPERIMENTALE

### Préparation d'une composition selon l'invention comprenant 45% d'eau de mer (SCHEM. 1) :

45 Kilogrammes d'une eau de mer prélevée en Bretagne par pompage sont filtrées à l'aide d'une trémie. L'eau de mer ainsi prélevée est entreposée dans un bassin de prétraitement. On procède ensuite au pompage de cette eau de mer à l'aide d'une pompe.

L'eau de mer prétraitée est injectée dans les canalisations d'un ensemble de filtration 1 représenté sur le schéma 1. L'ensemble de filtration 1 comprend une arrivée 2 de l'eau de mer. Cette arrivée d'eau 2 est connectée à un débitmètre 3 qui est branché sur la partie basse du carter 4 d'un préfiltre 5 (1 µm). La sortie du préfiltre 5 (toujours en partie basse du carter 4) est branchée sur la partie basse du carter 6 d'un filtre 7 (0.22 µm) dont la sortie est reliée à une canalisation qui sert à collecter l'eau issue de la filtration pour la stocker dans un container 8.

On complète ensuite la solution filtrée avec de l'eau purifiée et on ajoute l'extrait d'algue.

### I. Test de l'efficacité immunologique de la composition par l'étude de divers marqueurs des défenses immunitaires

Différents extraits d'algues, riches en fucanes de différents poids moléculaires, ont été comparés.

### • Evaluation de la sécrétion du peptide antimicrobien β-défensine-2

Trois extraits *d'Ascophyllum nodosum* ont été testés : l'un riche en fucanes de 4000 Da (Fuc 4000, bâton noir sur les diagrammes 1 et 2), un autre riche en fucanes de 8000 Da (Fuc 8000, bâton gris sur les diagrammes 1 et 2) et un dernier riche en fucanes de 20000 Da (Fuc 20000, bâton blanc sur les diagrammes 1 et 2). Un extrait de *Fucus vesiculosus* (Fuc Fucus) et un extrait d'*Undaria pinnatifida* (Fuc Undaria), dont le poids moléculaire des fucanes est compris entre 5000 et 30000 Da, ont également été testés. Ces échantillons sont solubilisés soit dans le milieu de culture RPMI contenant 2,5% de sérum de veau foetal, 0,5% de pénicilline/streptomycine et 1% de glutamine, soit dans l'eau de mer à 45% (Sal 15) à trois concentrations (1µg/mL, 100µg/mL et 1mg/mL). Les échantillons sont incubés avec les macrophages pendant 1h puis les surnageants sont récupérés pour dosage du peptide antimicrobien β-defensine-2.

Le relargage de β-defensine-2 est mesuré sur les macrophages par la technique ELISA (Enzyme-Linked Immunosorbent Assay). Cette technique permet le dosage de protéines dans un échantillon ; dans notre cas, la technique ELISA nous permet de quantifier le peptide β-defensine-2, libéré dans le surnageant cellulaire par les macrophages. L'antigène spécifique de la β-defensine-2 est accroché à un support, une microplaque 96 puits. On y dépose le surnageant cellulaire et la β-defensine-2 peut se lier à son antigène. Un anticorps primaire permet de reconnaître la β-defensine-2, puis un anticorps secondaire conjugué à une enzyme se lie à l'anticorps primaire. Une réaction enzymatique colorimétrique permet la révélation du complexe anticorps. L'appareil utilisé pour quantifier l'absorbance est un cytomètre adapté aux microplaques (Safire, TECAN).

La sécrétion de β-defensine-2 a été évaluée en mesurant le facteur de sécrétion qui est reporté en ordonnées sur les diagrammes 1 et 2 :
L'extrait d'Ascophyllum nodosum riche en fucanes de poids moléculaire 8000 Da (Fuc 8000) est celui qui est le plus efficace pour stimuler la sécrétion du peptide antimicrobien β-defensine-2.

Ces tests démontrent sans équivoque la synergie d'action entre les fucanes et l'eau de mer filtrée dans la stimulation du système immunitaire inné (à 1µg/mL : x0,97 dans le milieu de culture versus x1,18 dans l'eau de mer, à 100µg/mL : x1,26 versus x1,98 et à 1mg/mL : x1,28 versus x1,36).

### • Evaluation de la réponse inflammatoire avec TNF-α

Le relargage de cytokines pro-inflammatoires (TNF-α) est mesuré sur les macrophages par la technique ELISA, expliquée précédemment.

Un extrait d'*Ascophyllum nodosum* riche en fucanes de poids moléculaire moyen de 10000 Da (Ascophyscient) et un extrait de *Laminaria digitata* riche en fucanes de poids moléculaire moyen de 520000 Da (Fuc3 F) ont été testés pour leur capacité à réduire la sécrétion de cytokines pro-inflammatoires (TNF-α) induite par un agent bactérien (bâtons blancs sur les diagrammes 3 et 4, versus les mêmes échantillons sans LPS : bâtons noirs), le LPS d'Escherichia Coli pendant 24 heures à 37°C. Ils sont solubilisés soit dans le milieu de culture RPMI contenant 2,5% de sérum de veau foetal, 0,5% de pénicilline/streptomycine et 1% de glutamine, soit dans l'eau de mer à 45% aux concentrations indiquées sur les diagrammes 3 et 4, et incubés avec les macrophages.

La sécrétion de cytokines pro-inflammatoires a été mesurée par le dosage de TNF-α qui est montré sur les diagrammes 3 et 4, avec l'échelle en ordonnées représentée en unités arbitraires :
- Comme cela se voit à l'interprétation des résultats du diagramme 3, l'extrait de *Laminaria digitata* (Fuc3 F) ne stimule pas la production de cytokine proinflammatoire TNF-α. Il n'est pas capable de bloquer la production de cytokine induite par le LPS de Escherichia Coli. Il n'a donc pas d'effet anti-inflammatoire par rapport au TNF-α.
- En revanche, si le même dosage est effectué après incubation des macrophages avec la composition contenant 0.1% d'un extrait d'*Ascophyllum nodosum* (Ascophyscient) riche en fucanes de poids moléculaire moyen 10 kDa dissout dans 45% d'eau de mer filtrée (composition *Stimmuno_A*)*,* dans ce cas, la sécrétion de TNF-α induite par le LPS est bloquée (diagramme 4). L'extrait d'*Ascophyllum nodosum* riche en fucanes de poids moléculaire moyen de 10000 Da (Ascophyscient) en combinaison avec 45% de l'eau de mer filtrée a donc un fort effet anti-inflammatoire.

### • Evaluation de la réponse inflammatoire avec IL-1β

La technique ELISA est également employée ici.

Pour confirmer le contrôle de la réaction inflammatoire mesurée avec TNF-α par la formulation, le dosage d'une autre cytokine pro-inflammatoire, l'IL-1β, a donc été réalisé.

Comme cela se voit à l'interprétation des résultats illustrés sur le diagramme 5 qui représente le dosage d'IL-1β (unités arbitraires en ordonnées), l'extrait d'*Ascophyllum nodosum* (Ascophyscient) dissout dans 45% d'eau de mer filtrée (composition *Stimmuno_A*) ne stimule pas la production de cytokine proinflammatoire IL-1β. En conséquence, la composition *Stimmuno_A* selon l'invention avec 0.1% d'*Ascophyllum nodosum et* 45% d'eau de mer filtrée est capable de bloquer la production de cytokines induite par le LPS de Escherichia Coli. Ce qui traduit un contrôle de la sécrétion d'IL-1β en réponse à un stress bactérien.

Conclusion : *l'Ascophyllum nodosum* est efficace pour diminuer la production de cytokines proinflammatoires. L'extrait d'*Ascophyllum nodosum* riche en fucanes de poids moléculaire de 10000 Da contrôle la réponse inflammatoire contrairement à l'extrait de *Laminaria digitata* riche en fucanes de 520000 Da.

D'autres extraits d'algues et notamment de *Fucus vesiculosus* et d'Undaria *pinnatifida* dans de l'eau de mer filtrée ont également été testés, et ont permis de conclure que les meilleurs résultats ont été obtenus avec *Ascophyllum nodosum.*

### • Evaluation de l'autophagie et mise en évidence de la synergie d'action

D'autres essais (partie A. et B.) ont également été réalisés, pour mettre en évidence l'efficacité de la composition vis-à-vis de marqueurs de défense cellulaire qui ont été étudiés après incubation de cellules humaines avec des compositions contenant 0.1 - 0.2% d'un extrait d'*Ascophyllum nodosum* (Ascophyscient) riche en fucanes de poids moléculaire moyen 10 kDa dissout dans 45% d'eau de mer filtrée et complétée à 100% d'eau purifiée (qsp) : l'autophagie et la sécrétion du peptide antimicrobien β-défensine-2.

### Partie A.

La composition *Stimmuno_A* (0.1% (m/v) d'un extrait d'*Ascophyllum nodosum* (Ascophyscient) riche en fucanes de poids moléculaire moyen 10 kDa dissout dans 45% d'eau de mer filtrée et complétée à 100% d'eau purifiée (qsp)) est mise en contact avec des cellules du système immunitaire (macrophages humains) pendant 1h, la formulation est éliminée puis l'induction de l'autophagie est mesurée immédiatement ou 24h après. L'autophagie est un processus catabolique qui permet de maintenir l'homéostasie cellulaire. Elle est activée en cas de stress cellulaire pour la digestion des éléments pathogènes. En parallèle, les surnageants cellulaires sont récupérés pour y doser le peptide antimicrobien β-défensine-2 que les macrophages sécrètent pour éliminer les micro-organismes.

Les résultats obtenus sont présentés sur le diagramme 6 qui illustre la mesure de l'autophagie sur macrophage par cytométrie (test monodansylcadavérine, MDC). Lors de sa formation, l'autophagosome - lequel consiste en un compartiment de séquestration à double membrane généré à partir d'un phagosome - incorpore dans sa membrane des composants cytoplasmiques, dont la MDC, fluorescente. Après incubation avec les échantillons, les surnageants sont prélevés pour un dosage ultérieur du peptide β-défensine-2 et les cellules sont incubées avec une solution de MDC à 0,05mM (solution mère à préparer dans le DMSO à 50mM) dans du tampon PBS pendant 30 minutes à 37°C. Après incubation, la sonde est éliminée et les cellules sont rincées avec du PBS. La microplaque est ensuite lue par le cytofluorimètre Safire-TECAN aux longueurs d'onde de la MDC (Àexcitation = 380 nm, λémission = 525 nm).

Les bâtons gris plein de gauche quantifient l'effet immédiat mesuré après 1h d'incubation, les bâtons hachurés de droite quantifient l'effet retardé après 1+24h d'incubation. L'échantillon 1 correspond au contrôle négatif, l'échantillon 2 (au milieu du diagramme) correspond au test réalisé sur la composition *Stimmuno_A,* et l'échantillon 3 au test réalisé sur un contrôle positif. Le contrôle positif utilisé ici pour l'induction de l'autophagie est un agent bactérien LPS d'Escherichia Coli à 0,5µg/mL.

L'échelle en ordonnée correspond aux valeurs mesurées du facteur de modulation par rapport au contrôle négatif.

Les trois astérisques « *** » sur le diagramme 6 qui figurent sur les bâtons respectivement pour les échantillons 2 et 3 correspondent à une significativité statistique de p<0,001 (probabilité que la différence soit due au hasard) comparé au contrôle négatif (ANOVA, test Dunnett, logiciel GraphPad Prism 6).

Conclusion : Après incubation (1h) avec la composition *Stimmuno_A,* l'autophagie est significativement augmentée dans les macrophages que ce soit immédiatement après élimination de la composition ou 24h après. La composition *Stimmuno_A* permet de stimuler l'autophagie, un processus catabolique qui permet de maintenir l'homéostasie cellulaire via la digestion cellulaire, en prévision d'une agression par des éléments pathogènes.

### Partie B.

Des tests comparatifs ont été réalisés entre :
- une composition selon l'invention, la composition *Stimmuno_B* (0.2% (m/v) d'un extrait d'*Ascophyllum nodosum* (Ascophyscient) riche en fucanes de poids moléculaire moyen 10 kDa dissout dans 45% d'eau de mer filtrée et complétée à 100% d'eau purifiée (qsp)) ;
- une composition AN : comportant uniquement 0.2% (m/v) d'un extrait d'*Ascophyllum nodosum* (Ascophyscient) dissoute dans le tampon PBS (solution tampon phosphate salin, couramment utilisé en culture cellulaire); et
- une composition EDM : comportant de l'eau de mer filtrée titrant à 15g/L en NaCl.

Les résultats sont montrés sur le diagramme 7 qui compile les mesures de la sécrétion du peptide antimicrobien β-défensine-2 par les macrophages (technique ELISA) ; l'échelle en ordonnée correspond aux valeurs mesurées du facteur de modulation de la sécrétion par rapport au tampon PBS seul : l'échantillon 1 correspond au PBS seul, l'échantillon 2 (au milieu du diagramme) correspond au test réalisé sur la composition *Stimmuno_B,* l'échantillon 3 au test réalisé sur la composition AN, et l'échantillon 4 au test réalisé sur la composition EDM.

Après incubation (1h) avec la composition *Stimmuno_B* la sécrétion par les macrophages du peptide antimicrobien β-défensine-2 est augmentée 24h après. En revanche, la composition AN, ainsi que la composition EDM n'ont pas d'effet sur la sécrétion du peptide antimicrobien β-défensine-2. La composition *Stimmuno_B* permet de stimuler la sécrétion du peptide antimicrobien β-défensine-2, en prévision d'une agression par des éléments pathogènes.

Conclusion : Il y a donc une synergie d'action entre les composants de l'eau de mer filtrée et les polysaccharides sulfatés.

### • Evaluation de l'activité sur différents modèles avec différents agents de stress : marqueurs de l'immunité innée

Une composition *Stimmuno_C* selon l'invention a également été testée sur différents modèles cellulaires avec différents agents de stress selon le même protocole : le produit est mis en contact avec les cellules pendant un temps court (1h) puis les compositions sont éliminées et les cellules sont stressées par l'ajout d'un agent toxique pathogène. Au moment du stress, les cellules ne sont donc plus en contact avec la composition, cependant la réaction inflammatoire est significativement inhibée : ceci signifie que les cellules ont métabolisé et mémorisé les signaux déclenchés par la composition *Stimmuno_C* pour pouvoir ainsi contrôler la réponse inflammatoire.

Composition *Stimmuno_C :*
- Solution de 253 kg préparée avec 116.4 kg d'eau de mer filtrée, 269.5 g d'extrait d'Ascophyllum nodosum riche en fucanes (Ascophyscient), 136 g d'eau purifiée.
- Les caractéristiques de la solution obtenue sont:
   PH : 7.2
   Osmolalité : 471 mosmol/Kg
   Chlorure exprimés en NaCl : 14.6 g/l
   Sucres totaux : 0.395 g/l (ou oses totaux mesurés par la méthode de Dubois et a. (1956))

Des cellules immunitaires (macrophages) ont été incubées pendant 1h avec d'une part, la composition *Stimmuno_C,* et d'autre part du milieu de culture (MC 0% = témoin négatif) puis les solutions ont été éliminées et les cellules incubées avec du LPS d'Escherichia Coli pour induire un stress inflammatoire bactérien.

Deux cytokines ont été dosées par le test ELISA dans les surnageants cellulaires : une cytokine pro inflammatoire, l'IL-1β (bâtons noirs sur le diagramme 8) et une cytokine anti-inflammatoire, l'IL-10 (bâtons blanc sur le diagramme 8).

Les résultats sont montrés sur le diagramme 8 qui compile les dosages de cytokines après 1h d'incubation suivie de 24h dans le LPS d'Escherichia Coli sur macrophage. L'échelle en ordonnée correspond aux facteurs amplificateurs de sécrétion. La ligne noire en pointillés représente le niveau basal de sécrétion sans LPS qui est de 1.

Lorsque les cellules ont été incubées avec le milieu de culture-témoin négatif (à gauche sur le diagramme), le LPS stimule la sécrétion d'IL-1β d'un facteur 2,7 et celle d'IL-10 d'un facteur 4,2 par rapport au niveau basal de sécrétion sans LPS. Les cellules répondent ainsi au LPS en sécrétant un signal d'attaque (l'IL-1β) et un signal de défense (l'IL-10). Lorsque les cellules ont été incubées avec la composition *Stimmuno_C* (à droite sur le diagramme 8), le LPS stimule la sécrétion d'IL-1β d'un facteur 1,3 et celle d'IL-10 d'un facteur 5,8. Par conséquent, la composition *Stimmuno_C* diminue le signal d'attaque tout en stimulant le signal de défense induit par le LPS.

### Conclusion :

La réaction inflammatoire est significativement inhibée : ceci signifie que les cellules ont métabolisé et mémorisé (effet mémoire) les signaux déclenchés par la composition *Stimmuno_C* pour pouvoir ainsi contrôler la réponse inflammatoire. Ces résultats expérimentaux démontrent la stimulation de l'immunité innée (effet mémoire) par la composition selon l'invention.

### • Evaluation de l'activité sur des cellules épithéliales pulmonaires humaines infectée par un virus

Des cellules épithéliales pulmonaires sont prélevées chez l'homme, et incubées en présence de la composition *Stimmuno_A.* Il est montré que la composition selon l'invention diminue drastiquement l'expression de gènes impliqués dans la réponse inflammatoire ainsi que la sécrétion de cytokines pro inflammatoires induite par le polylC, agent de stress viral.

Des cellules épithéliales bronchiques prélevées chez l'homme ont été incubées pendant 1h avec du NaCl 0,9% (utilisé ici comme témoin négatif) ou la composition *Stimmuno_A,* puis les solutions ont été éliminées et les cellules incubées avec du PolylC pour induire un stress inflammatoire viral.

L'expression du gène de la cytokine pro inflammatoire CCL20 a été dosée par RTqPCR. L'expression de CCL20 est surrégulée chez les patients atteints de rhinosinusite chronique.

Les résultats sont montrés sur le diagramme 9, qui quantifie l'expression du gène de la protéine CCL20 par les cellules épithéliales bronchiques. L'échelle en ordonnée rapporte les valeurs mesurées pour l'expression relative (2 delta Ct x 1000).

Le PolylC stimule la surexpression du gène CCL20 (barre orange), mais lorsque les cellules ont été incubées avec la composition *Stimmuno_A,* le PolylC stimule beaucoup moins la sécrétion de ce gène (diminution d'un facteur 25). Par conséquent, la composition *Stimmuno_A* diminue le signal d'attaque induit par le PolylC.

### Conclusion :

Les résultats montrent que sur les cellules épithéliales pulmonaires prélevées chez l'homme, la composition *Stimmuno_A* diminue drastiquement l'expression de gènes impliqués dans la réponse inflammatoire ainsi que la sécrétion de cytokines pro inflammatoires induite par le polylC, agent de stress viral.

En conclusion générale, la composition selon l'invention prépare les cellules de l'organisme à se défendre en cas d'attaque bactérienne ou virale. La composition bloque et contrôle la réponse inflammatoire que pourraient induire diverses agressions extérieures. Cette défense s'applique aux différents points d'entrée dans l'organisme : muqueuse nasale, muqueuse buccale, gorge, oeil, peau, muqueuse vaginale...

### II. Tests d'innocuité vis-à-vis des cellules de l'organisme

La bonne tolérance de la composition contenant 0.1% d'un extrait d'*Ascophyllum nodosum* (Ascophyscient) riche en fucanes de poids moléculaire moyen de 10 kDa dissout dans 45% d'eau de mer filtrée (composition *Stimmuno_A*) pour l'épithélium nasal a été confirmée par l'étude de la morphologie des cellules ciliées de l'épithélium nasal. Les cils vibratiles de ces cellules permettent d'éliminer le mucus. Le maintien de la morphologie ciliaire est essentiel au bon fonctionnement de l'épithélium nasal.

Protocole : Des cellules humaines de l'épithélium nasal ont été incubées soit avec du chlorure de sodium isotonique à 0.9%, soit avec la composition pendant 1h. Le cytosquelette des cellules a été coloré par un marqueur fluorescent (vert) et les noyaux par un autre marqueur fluorescent (bleu).

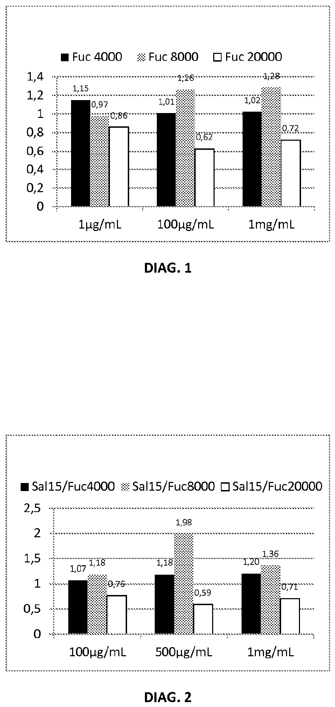

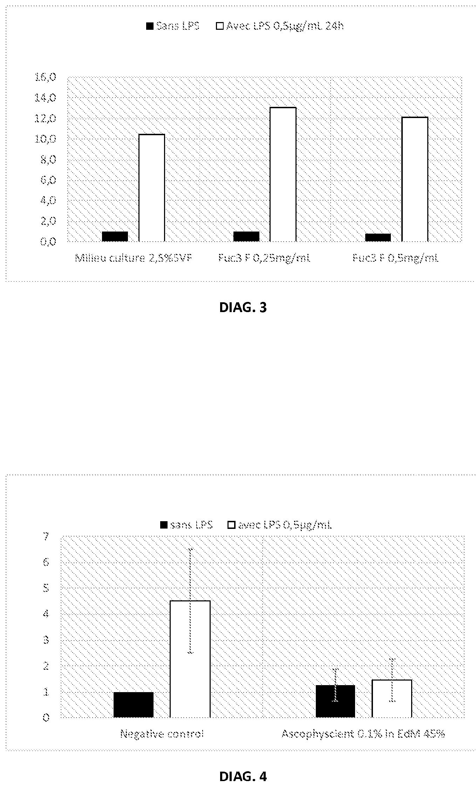

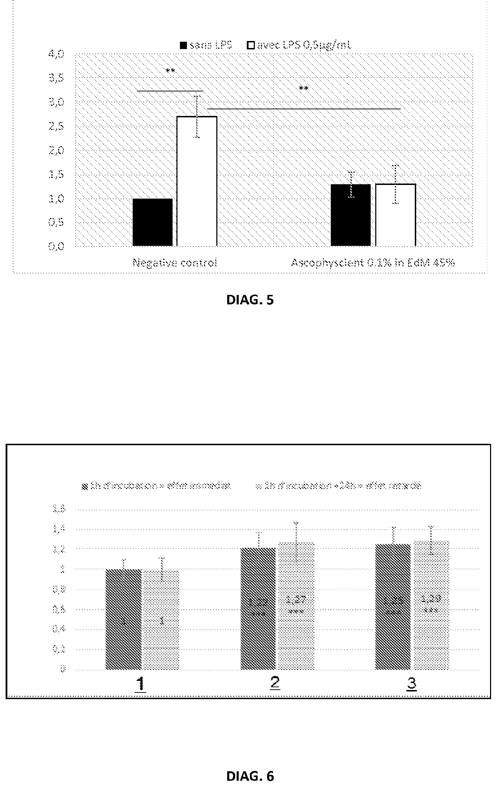

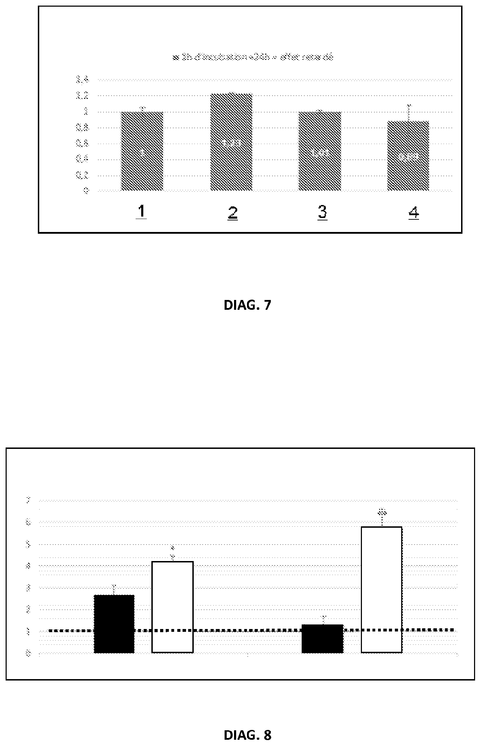

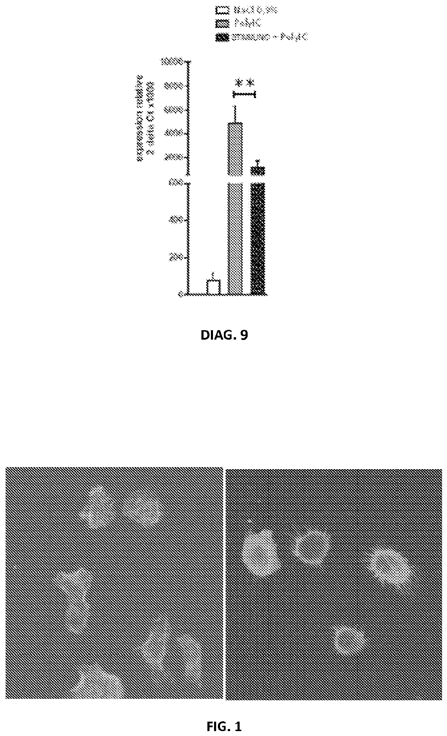

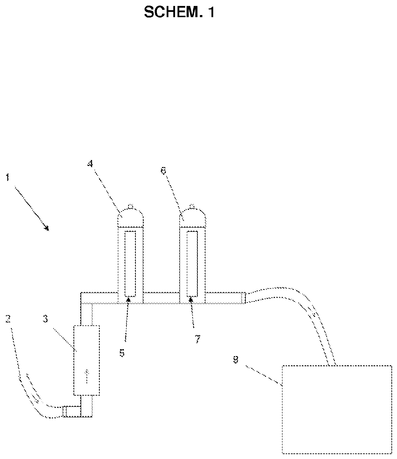

La figure 1 montre que les cils sont beaucoup plus visibles et plus longs lorsque les cellules épithéliales ciliées sont incubées avec la composition selon l'invention (montré sur la photo de droite) par rapport à la solution de chlorure de sodium isotonique (montré sur la photo de gauche).

Il peut donc être conclu que la composition contenant l'extrait d'*Ascophyllum nodosum* et 45% d'eau de mer filtrée est donc parfaitement tolérée par l'épithélium nasal humain.

## Revendications

1. Composition aqueuse pour son utilisation dans la prévention d'au moins une des maladies provoquées par l'invasion d'au moins un agent pathogène par au moins un des éléments sélectionnés parmi la peau, l'œil et une muqueuse, comprenant 20 à 60% en poids d'eau de mer filtrée par rapport à la masse totale de la composition finale obtenue et comprenant un polysaccharide sulfaté qui est un fucane ayant une masse de 2 à 21 kDa et provenant de l'algue brune *Ascophyllum nodosum,* sous forme de cette algue entière ou d'une partie de cette dernière, ou sous forme d'extrait de ladite algue, ou encore sous forme d'une molécule de synthèse identique à un polysaccharide sulfaté naturel contenu dans l'algue, **caractérisée en ce que** la composition stimule le système immunitaire en stimulant l'immunité innée.

2. Composition aqueuse pour son utilisation selon la revendication 1, comprenant au moins une eau sélectionnée parmi de l'eau purifiée, de l'eau distillée et de l'eau déminéralisée dans une teneur de 40 à 80% en poids par rapport à la masse totale de la composition finale obtenue.

3. Composition aqueuse pour son utilisation selon l'une des revendications 1 à 2, dans laquelle le polysaccharide sulfaté est avantageusement pris dans une teneur de 0.01 à 0.5% en poids par rapport à la masse totale de la composition finale obtenue.

4. Composition aqueuse pour son utilisation selon l'une des revendications 1 à 3, dans laquelle la concentration des éléments dans la composition finale est telle que :
Cl (g/L) 5 - 15
Na (g/L) 2.5 - 7
Mg (g/L) 0.45 - 0.70
Ca (g/L) 0.10 - 0.30
K (g/L) 0.10 - 0.30

5. Composition aqueuse pour son utilisation selon l'une des revendications 1 à 4, dans laquelle la concentration des éléments et des substances dans la composition finale est telle que :
CI (g/L) 7.9 - 10.1
Na (g/L) 4.3 - 5.3
Mg (g/L) 0.51 - 0.63
Ca (g/L) 0.16 - 0.22
K (g/L) 0.15 - 0.23
Sucres totaux (g/L) 0.25 - 0.55

6. Composition aqueuse pour son utilisation selon l'une des revendications 1 à 5, dans laquelle le pH est de 6.5 à 8.5.

7. Composition aqueuse pour son utilisation selon l'une des revendications 1 à 6, dans laquelle l'osmolarité à 25° C est de 400 à 550 mOsmol/Kg.

8. Composition pour son utilisation selon l'une des revendications 1 à 7, dans laquelle ladite muqueuse est sélectionnée parmi la muqueuse nasale, la muqueuse buccale et la muqueuse vaginale.

9. Composition pour son utilisation selon l'une des revendications 1 à 8, **caractérisée en ce qu'**elle prévient les états inflammatoires des muqueuses.

## Patentansprüche

1. Wässrige Zusammensetzung für ihre Verwendung zur Vorbeugung von mindestens einer der Krankheiten, die von der Invasion mindestens eines pathogenen Stoffs in mindestens eins der Elemente, die aus der Haut, dem Auge und einer Schleimhaut ausgewählt sind, hervorgerufen werden, umfassend 20 bis 60 Gew.-% gefiltertes Meerwasser im Verhältnis zum Gesamtgewicht der erhaltenen endgültigen Zusammensetzung und umfassend ein sulfatiertes Polysaccharid, das ein Fucan ist, mit einer Masse von 2 bis 21 kDa und von der Alge *Ascophyllum nodosum* stammt, in Form dieser ganzen Alge oder eines Teils derselben oder in Form eines Extrakts der Alge oder auch in Form eines Synthesemoleküls, das mit einem natürlichen sulfatierten Polysaccharid, das in der Alge enthalten ist, identisch ist, **dadurch gekennzeichnet, dass** die Zusammensetzung das Immunsystem durch Stimulation des angeborenen Immunsystems stimuliert.

2. Wässrige Zusammensetzung für ihre Verwendung nach Anspruch 1, umfassend mindestens ein Wasser, das aus dem gereinigten Wasser, dem destillierten Wasser und dem demineralisierten Wasser ausgewählt ist, in einem Gehalt von 40 bis 80 Gew.-% im Verhältnis zur Gesamtmasse der erhaltenen endgültigen Zusammensetzung.

3. Wässrige Zusammensetzung für ihre Verwendung nach einem der Ansprüche 1 bis 2, wobei das sulfatierte Polysaccharid in vorteilhafter Weise in einem Gehalt von 0,01 bis 0,5 Gew.-% im Verhältnis zur Gesamtmasse der erhaltenen endgültigen Zusammensetzung herangezogen ist.

4. Wässrige Zusammensetzung für ihre Verwendung nach einem der Ansprüche 1 bis 3, wobei die Konzentration der Elemente in der endgültigen Zusammensetzung folgendermaßen ist:
Cl (g/L) 5 - 15
Na (g/L) 2,5 - 7
Mg (g/L) 0,45 - 0,70
Ca (g/L) 0,10 - 0,30
K (g/L) 0,10 - 0,30

5. Wässrige Zusammensetzung für ihre Verwendung nach einem der Ansprüche 1 bis 4, wobei die Konzentration der Elemente und der Substanzen in der endgültigen Zusammensetzung folgendermaßen ist:
Cl (g/L) 7,9 - 10,1
Na (g/L) 4,3 - 5,3
Mg (g/L) 0,51 - 0,63
Ca (g/L) 0,16 - 0,22
K (g/L) 0,15 - 0,23
Zucker insgesamt (g/L) 0,25 - 0,55

6. Wässrige Zusammensetzung für ihre Verwendung nach einem der Ansprüche 1 bis 5, wobei der pH 6,5 bis 8,5 beträgt.

7. Wässrige Zusammensetzung für ihre Verwendung nach einem der Ansprüche 1 bis 6, wobei die Osmolarität bei 25 °C 400 bis 550 mOsmol/kg beträgt.

8. Wässrige Zusammensetzung für ihre Verwendung nach einem der Ansprüche 1 bis 7, wobei die Schleimhaut aus der Nasenschleimhaut, der Mundschleimhaut und der vaginalen Schleimhaut ausgewählt ist.

9. Wässrige Zusammensetzung für ihre Verwendung nach einem der Ansprüche 1 bis 8, **dadurch gekennzeichnet, dass** sie entzündlichen Zuständen der Schleimhäute vorbeugt.

## Claims

1. Aqueous composition for use in the prevention of at least one of the diseases provoked by the invasion of at least one pathogenic agent by at least one of the elements selected from the skin, the eye and a mucous membrane, comprising from 20 to 60% by weight of filtered seawater relative to the total mass of the final composition and comprising a sulphated polysaccharide which is a fucan having a mass from 2 to 21 kDa and originating from the brown seaweed *Ascophyllum nodosum,* in the form of this whole seaweed or part of this seaweed, or in the form of an extract of said seaweed, or in the form of a synthetic molecule identical to a natural sulphated polysaccharide contained in the seaweed, **characterized in that** the composition stimulates the immune system by stimulating the innate immunity.

2. Aqueous composition for its use according to claim 1, comprising at least one water selected from purified water, distilled water and demineralised water in a content from 40 to 80% by weight relative to the total mass of the final composition obtained.

3. Aqueous composition for its use according to one of claims 1 to 2, wherein the sulphated polysaccharide is advantageously taken in a content from 0.01 to 0.5% by weight relative to the total mass of the final composition obtained.

4. Aqueous composition for its use according to one of claims 1 to 3, wherein the concentration of the elements in the final composition is such that:
Cl (g/L) 5 - 15
Na (g/L) 2.5 - 7
Mg (g/L) 0.45 - 0.70
Ca (g/L) 0.10 - 0.30
K (g/L) 0.10 - 0.30

5. Aqueous composition for its use according to one of claims 1 to 4, wherein the concentration of the elements and of the substances in the final composition is such that:
Cl (g/L) 7.9 - 10.1
Na (g/L) 4.3 - 5.3
Mg (g/L) 0.51 - 0.63
Ca (g/L) 0.16 - 0.22
K (g/L) 0.15 - 0.23
Total sugars (g/L) 0.25 - 0.55

6. Aqueous composition for its use according to one of claims 1 to 5, wherein the pH is from 6.5 to 8.5.

7. Aqueous composition for its use according to one of claims 1 to 6, wherein the osmolarity at 25° C is from 400 to 550 mOsmol/Kg.

8. Composition for its use according to one of claims 1 to 7, wherein said mucous membrane is selected from the nasal mucous membrane, oral mucous membrane and the vaginal mucous membrane.

9. Composition for its use according to one of claims 1 to 8, for the prevention of inflammatory states of the mucous membranes.
